# EUROPÄISCHE PATENTSCHRIFT

(11) **EP 0 975 288 B1**
(45) Veröffentlichungstag und Bekanntmachung des Hinweises auf die Patenterteilung: **02.10.2002**
(21) Anmeldenummer: 97915251.9
(22) Anmeldetag: 15.04.1997
(51) Int. Cl.: A61F 2/44

(54) **TELESKOPIERENDE WIRBELPROTHESE**
TELESCOPIC VERTEBRAL PROSTHESIS
PROTHESE DE VERTEBRE TELESCOPIQUE

(43) Veröffentlichungstag der Anmeldung: 02.02.2000
(73) Patentinhaber: Synthes AG Chur, 7002 Chur (CH)
(72) Erfinder: SCHÄR, Manuel, CH-4132 Muttenz (CH); HATEBUR, Alex, CH-4056 Basel (CH); SCHLÄPFER, Fridolin, CH-8750 Glarus (CH)
(74) Vertreter: Lusuardi, Werther Giovanni, Dr.
(86) Internationale Anmeldenummer: CH9700151
(87) Internationale Veröffentlichungsnummer: WO98046173

(56) Entgegenhaltungen:
- EP-A- 0 490 159
- WO-A-92/01428
- WO-A-94/18913
- DE-A- 3 741 487
- DE-A- 19 604 246
- DE-C- 4 012 622
- DE-C- 19 500 170
- US-A- 4 554 914

## Beschreibung

Die Erfindung bezieht sich auf eine Vorrichtung zum Ersatz von Wirbelkörpern aus der menschlichen Wirbelsäule gemäss dem Oberbegriff des Patentanspruchs 1.

Wird ein Wirbelkörper krank oder erleidet einen Defekt muss dieser aus der Wirbelsäule entfernt werden. Aus dem Stand der Technik sind einige distanzhaltende Implantate zum Ersatz des fehlenden Wirbelkörpers bekannt. Das Implantat umfasst gegeneinander verschiebbare Teile, die unter anderem mittels Rasterungen eine Einstellung der Länge des Implantates gestatten, und zwei spezielle Endplatten, die zur Verankerung des Implantates in den anschliessenden intakten Wirbelkörpern dienen. Solche Wirbelprothesen oder Implantate sind beispielsweise aus der AT R 24426 RASHEED oder aus der DE 196 04 246 A1 JEANSON bekannt.

Der Nachteil der bekannten Wirbelprothesen besteht darin, dass die beiden gegeneinander verschiebbaren Teile keine geschlossenen Hohlzylinder sind und daher die gesamte Wirbelprothese eine geringe Steifigkeit aufweist.

Der Erfindung liegt die Aufgabe zugrunde, mit einer stabilen und längeneinstellbaren Wirbelprothese die biomechanischen und physiologischen Eigenschaften der Wirbelsäule trotz des entfernten Wirbelkörpers zu erhalten. Grosse Bedeutung gilt daneben auch der Handhabung des Wirbelsäulenimplantates im Lauf der Operation.

Die Erfindung löst die gestellte Aufgabe mit einer Vorrichtung, welche die Merkmale des Anspruchs 1 aufweist.

Eine Ausführung der erfindungsgemässen Vorrichtung umfasst zwei ineinander verschiebbare Hohlzylinder. Der innere Hohlzylinder ist am äusseren Umfang mit einer Rasterung versehen. Der äussere Hohlzylinder weist mindestens ein federndes Element auf, das in die Rasterung des inneren Hohlzylinders einklinkt und die Länge der Vorrichtung festlegt. Durch die Rasterung und das einklinkbare, federnde Element kann die erfindungsgemässe Vorrichtung verlängert werden und bei der Operation eingepasst werden.

Eine andere Ausführung der erfindungsgemässen Vorrichtung umfasst zwei ineinander koaxial angeordnete und entlang dieser Achse gegeneinander verschiebbare Hohlkörper, eine in einer im äusseren Hohlkörper angebrachten Nute gelagerten Feder und Endplatten, die zur Verankerung der Vorrichtung in den anschliessenden Wirbelkörpern dienen. Der innere Hohlkörper ist an seiner äusseren Mantelfläche mit einer Rasterung versehen. Die Feder weist an ihrer Innenfläche eine Rasterung auf, welche beim Einklinken in die Rasterung am inneren Hohlkörper diesen in axialer Richtung fixiert. Da die Feder von aussen durch eine Öffnung im äusseren Hohlkörper auch mit einem Hilfsinstrument gespreizt und aus der Rasterung gelöst werden kann, ist eine kontrollierte Kompression dieser Variante der erfindungsgemässen Vorrichtung im Bedarfsfall auch in situ möglich. Auch ist eine Ausführung mit drei ineinander verschiebbaren Hohlkörpern möglich, wobei die äusseren und inneren Zylinderflächen jeweils wie bei der beschriebenen, zweiteiligen Variante gestaltet sind.

Die durch diese Variante der Erfindung erreichten Vorteile sind im wesentlichen darin zu sehen, dass bei der erfindungsgemässen Vorrichtung eine Feder mit einer Rasterung zur Fixierung der Länge des Implantates eingebaut ist. Die Bauhöhe des Implantates kann somit verkleinert werden und bei kleinen Freiräumen zwischen den angrenzenden Wirbeln eingesetzt werden, was eine Anwendung der Prothese auch in der Nähe der Halswirbelsäule gestattet. Durch Spreizen der Feder kann die erfindungsgemässe Wirbelprothese wieder komprimiert und entfernt werden. Ausserdem können die beiden Hohlzylinder relativ dickwandig sein und somit eine sehr stabile Prothese bilden.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch den inneren Hohlkörper 1 der erfindungsgemässen Vorrichtung;
Fig. 2 einen Längsschnitt durch den äusseren Hohlkörper 2 einer Variante der erfindungsgemässen Vorrichtung;
Fig. 3 einen Längsschnitt durch eine weitere Ausführung der erfindungsgemässen Vorrichtung;
Fig. 4 eine perspektivische Darstellung der ringförmigen Feder 13 gemäss einer Ausführung der erfindungsgemässen Vorrichtung;
Fig. 5 eine perspektivische Darstellung der ringförmigen Feder (13) mit einem Ausschnitt aus dem äusseren Hohlkörper gemäss einer Ausführung der erfindungsgemässen Vorrichtung;
Fig. 6 einen perspektivischen Ausschnitt der beiden Hohlkörper 1,2 und eines federnden Bügels 24 gemäss einer Ausführung der erfindungsgemässen Vorrichtung; und
Fig. 7 eine weitere Ausführung des inneren Hohlkörpers 1 der erfindungsgemässen Vorrichtung.

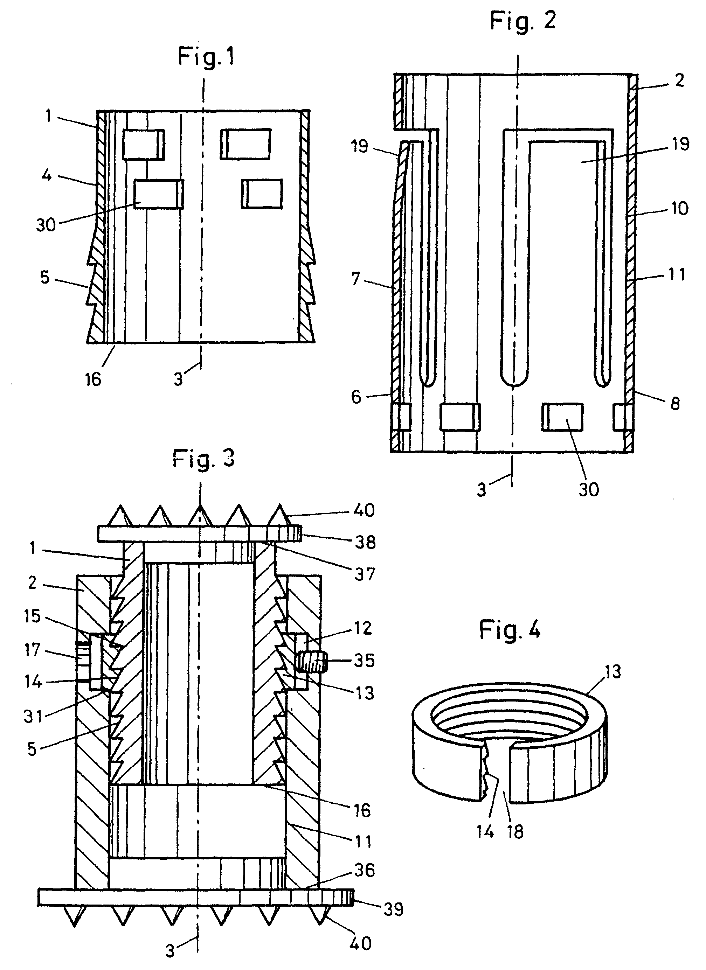

Fig. 1 zeigt den inneren Hohlzylinder 1 gemäss einer Ausführung der erfindungsgemässen Vorrichtung. Die Rasterung 5 auf der Mantelfläche ist nur auf einem Teil der Länge des inneren Hohlzylinders angebracht. Je nach Ausführungsart der erfindungsgemässen Vorrichtung ist der nicht mit der Rasterung 5 versehene Teil des inneren Hohlzylinders 1 mit radialen Durchgangsöffnungen 30 ausgestattet.

Der in Fig. 2 dargestellte äussere Hohlzylinder 2 entspricht einer Ausführung der erfindungsgemässen Vorrichtung, die als federndes Element 7 mehrere Zungen 19 aufweist, welche aus der Wand 6 des äusseren Hohlzylinders 2 ausgeschnitten sind und gegen die Zentralachse 3 hin eingebogen sind. Je nach Ausführungsart der erfindungsgemässen Vorrichtung ist der äussere Hohlzylinder 2 mit radialen Durchgangsöffnungen 30 ausgestattet.

Die in Fig. 3 dargestellte Variante der erfindungsgemässen Vorrichtung zeigt den äusseren Hohlkörper 2, der innen koaxial zur Zentralachse 3 mit einer zylindrischen Bohrung 10, einer ebenfalls zylindrischen Nute 12 und einer von dieser Nute 12 zur äusseren Mantelfläche des äusseren Hohlkörpers 2 durchgehenden Öffnung 17 versehen ist. Im äusseren Hohlkörper 2 gleitbar und entlang der Zentralachse 3 gegen diesen verschiebbar angeordnet ist der innere Hohlkörper 1, welcher an seiner Aussenfläche mit einer Rasterung 5 versehen ist. In die Nute 12 ist eine Feder 13, die innen mit einer zur Rasterung 5 des inneren Hohlkörpers 1 formschlüssigen Rasterung 14 versehen ist, eingelegt. Wird die Feder 13 mit einem Hilfsmittel, das durch die Öffnung 17 im äusseren Hohlelement 2 in den Spalt 18 der Feder 13 bringbar ist, gespreizt, kann der innere Hohlkörper 1 relativ zum äusseren in beide Richtungen verschoben werden.

Eine weitere in Fig. 3 dargestellte Variante der erfindungsgemässen Vorrichtung ist im Bereich der Nute 12 mit einer den äusseren Hohlkörper 2 radial durchdringenden Verriegelungsschraube 35 zur Blockierung der beiden Hohlkörper 1,2 in Richtung der Zentralachse 3 ausgerüstet.

Ebenfalls in Fig. 3 enthalten sind Varianten der erfindungsgemässen Vorrichtung, die an den beiden freien Enden 36, 37 der Hohlkörper 1,2 mit Endplatten 38, 39, welche knochenseitig Dorne 40 aufweisen können, ausgerüstet.

Die ringförmige Feder 13 der in Fig. 3 dargestellten Ausführung der erfindungsgemässen Vorrichtung wird in Fig. 4 dargestellt. Durch einen Spalt 18 ist die Feder 13 am Umfang geöffnet. Eine Rasterung 14, die in die Rasterung 5 auf dem äusseren Umfang des inneren Hohlkörpers 1 einklinkbar ist, ist auf der inneren Mantelfläche der ringförmigen Feder 13 angebracht.

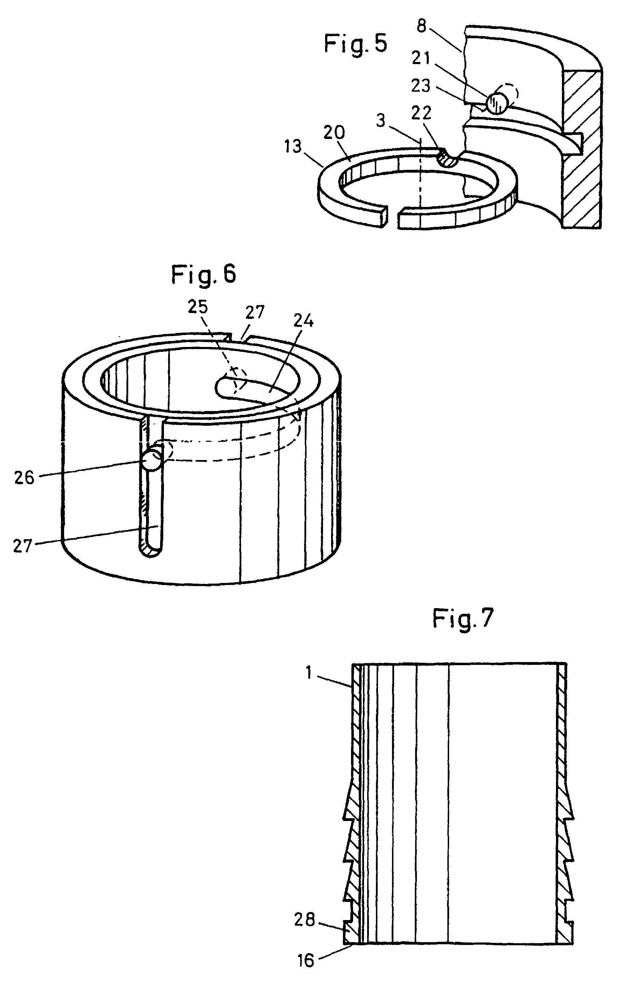

Fig. 5 zeigt eine weitere Ausführung der in Fig. 3 dargestellten Version der erfindungsgemässen Vorrichtung. Ein Stift 21 ist in eine radial durch den äusseren Hohlkörper 2 dringende Öffnung 23 eingesetzt, welcher in eine Kerbe 22 auf der oberen Stirnfläche 20 der Feder 13 einrastet und eine Verdrehung der Feder 13 um die Zentralachse 3 verhindert.

Fig. 6 zeigt eine weiter Ausführung der erfindungsgemässen Vorrichtung. Ein federnder Bügel 24 mit nach aussen gebogenen, freien Enden 25, 26 , die in Längsschlitze 27 am äusseren Hohlkörper 2 einrastbar sind, sichert die beiden Hohlkörper 1, 2 gegen Verdrehung.

In einer weiteren in Fig. 7 dargestellten Ausführung der in Fig. 3 dargestellten Version der erfindungsgemässen Vorrichtung ist der innere Hohlkörper 1 an seinem unteren Ende 16 mit einem Absatz 28 versehen, dessen Aussendurchmesser grösser als der Innendurchmesser der ungedehnten Feder 13 ist. Der Absatz 28 am unteren Ende 16 verhindert, dass der innere Hohlkörper 1 nach oben an der Feder 13 vorbeigleiten und aus dem äusseren Hohlkörper 2 herausrutschen kann.

## Patentansprüche

1. Vorrichtung zum Ersatz von Wirbelknochen, die einen inneren (1) und einen äusseren (2) Hohlkörper umfasst, welche entlang einer Zentralachse (3) ineinander koaxial gleitbar und in Richtung dieser Zentralachse (3) relativ zueinander verschiebbar sind und der innere Hohlkörper (1) auf seiner äusseren Mantelfläche (4) mit einer Rasterung (5) versehen ist, **dadurch gekennzeichnet, dass**
A) der äussere Hohlkörper (2) entlang der Zentralachse (3) durchgehend durchbohrt (10) ist;
B) der äussere Hohlkörper (2) mindestens ein federndes Element (7) aufweist, das in die Bohrung (10) hineinragt und den Durchmesser der Bohrung (10) so verringert, dass das federnde Element (7) in die Rasterung (5) am inneren Hohlkörper (1) einklinkt; und
C) der innere Hohlkörper (1) starr ist.

2. Vorrichtung nach Anspruch 1, **dadurch gekennzeichnet, dass** beide Hohlkörper (1,2) hohlzylindrisch sind.

3. Vorrichtung nach Anspruch 1 oder 2, **dadurch gekennzeichnet, dass** dieses mindestens eine federnde Element (7) als Zunge (19) aus der Wand (6) des äusseren Hohlkörpers (2) ausgeschnitten ist.

4. Vorrichtung nach Anspruch 1 oder 2, **dadurch gekennzeichnet, dass** als federndes Element (7) eine ringförmige, radial durch einen Spalt (18) geöffnete Feder (13), die mit einer Rasterung (14) am inneren Umfang (15) versehen ist und in einer an der inneren Mantelfläche (11) des äusseren Hohlkörpers (2) umlaufenden Nute (12) gelagert ist, in die Rasterung (5) am inneren Hohlkörper (1) einklinkt.

5. Vorrichtung nach Anspruch 4, **dadurch gekennzeichnet, dass** durch eine den äusseren Hohlkörper (2) radial durchdringende Öffnung (17) die Feder (13) mit einem Hilfsmittel, das durch die Öffnung (17) in den Spalt (18) der Feder (13) einbringbar ist, die Feder (13) gespreizt und damit die Rasterung (14) der Feder (13) aus der Rasterung (5) am inneren Hohlkörper (1) ausgeklinkt werden kann.

6. Vorrichtung nach einem der Ansprüche 4 oder 5, **dadurch gekennzeichnet, dass** die Feder (13) auf einer Stirnfläche (20) eine Kerbe (22) aufweist.

7. Vorrichtung nach einem der Ansprüche 4 bis 6, **dadurch gekennzeichnet, dass** ein Stift (21) in eine durch den äusseren Hohlkörper (2) dringende Öffnung (23) eingesetzt ist und dieser Stift (21) die Feder (13) durch Formschluss mit der Kerbe (22) gegen Drehung um die Zentralachse (3) sichert.

8. Vorrichtung nach einem der Ansprüche 1 bis 7, **dadurch gekennzeichnet, dass** ein federnder Bügel (24) mit nach aussen gebogenen, freie Enden (25,26) in Längsschlitzen (27) am äusseren Hohlkörper (2) einrastbar ist und ein Verdrehen der beiden Hohlkörper (1,2) verhindert.

9. Vorrichtung nach einem der Ansprüche 4 bis 8, **dadurch gekennzeichnet, dass** der innere Hohlkörper (1) an seinem unteren Ende (16) mit einem Absatz (28) versehen ist, dessen Aussendurchmesser grösser als der Innendurchmesser der ungedehnten Feder (13) ist und der verhindert, dass der innere Hohlkörper (1) nach oben an der Feder (13) vorbeigleiten und aus dem äusseren Hohlkörper (2) herausrutschen kann.

10. Vorrichtung nach einem der Ansprüche 1 bis 9, **dadurch gekennzeichnet, dass** beide Hohlkörper (1, 2) radial zur Zentralachse (3) Durchgangsöffnungen (30) aufweisen.

11. Vorrichtung nach einem der Ansprüche 4 bis 10, **dadurch gekennzeichnet, dass** die Nut (12) und die Feder (13) gegen die Berührungsfläche (31) hin sich verjüngend konisch gestaltet sind.

12. Vorrichtung nach einem der Ansprüche 1 bis 11, **dadurch gekennzeichnet, dass** einer oder beide der Hohlkörper (1,2) entlang der Zentralachse (3) abgekröpft ist oder sind.

13. Vorrichtung nach einem der Ansprüche 1 bis 12, **dadurch gekennzeichnet, dass** die beiden Hohlkörper (1, 2) einen nicht kreisförmigen Querschnitt haben.

14. Vorrichtung nach einem der Ansprüche 1 bis 13, **dadurch gekennzeichnet, dass** an den beiden freien Enden (36, 37) der beiden Hohlkörper (1, 2) Endplatten (38, 39) angebracht werden.

15. Vorrichtung gemäss Anspruch 14, **dadurch gekennzeichnet, dass** knochenseitig an den Endplatten (38, 39) Dorne (40) angebracht sind.

16. Vorrichtung nach einem der Ansprüche 1 bis 15, **dadurch gekennzeichnet, dass** im äusseren Hohlkörper (2) eine Verriegelungsschraube (35) eingeschraubt wird.

17. Vorrichtung nach einem der Ansprüche 4 bis 16, **dadurch gekennzeichnet, dass** die Rasterung (5;14) sowohl am inneren Hohlkörper (1) als auch an der Feder (13) auf der druckbelasteten Seite in Richtung des oberen Endes (37) hinterschnitten ist.

## Claims

1. Device for replacing vertebral bones, comprising an interior hollow body (1) and an exterior hollow body (2) which can slide into each other coaxially along a central axis (3) and can be moved in relation to one another in the direction of said central axis (3), and the interior hollow body (1) is provided with a catch mechanism (5) on its outer surface (4), **characterized in that**
A) the exterior hollow body (2) is bored through all the way along the central axis (3);
B) and the exterior hollow body (2) has at least one elastic element (7) which projects into the bore (10) and reduces the diameter of said bore (10) so that the elastic element (7) latches into the catch mechanism (5) of the interior hollow body (1); and
C) the interior hollow body (1) is rigid.

2. Device according to Claim 1, **characterized in that** both hollow bodies (1, 2) are hollow cylinders.

3. Device according to Claim 1 or 2, **characterized in that** this minimum of one elastic element (7) is cut as a tongue (19) out of the wall (6) of the exterior hollow body (2).

4. Device according to Claim 1 or 2, **characterized in that** a ring-shaped spring (13) which latches into the catch mechanism (5) on the interior hollow body (1) is opened radially through a gap (18), is provided with a catch mechanism (14) on the inside periphery (15) and is mounted in a groove (12) running on the inside surface (11) of the exterior hollow body (2).

5. Device according to Claim 4, **characterized in that** the spring (13) is spread through an opening (17) passing radially through the exterior hollow body (2) by using an auxiliary means which can be introduced into the gap (18) in the spring (13) through said opening (17), and thus the catch mechanism (14) of the spring (13) can be unlatched from the catch mechanism (5) on the interior hollow body (1).

6. Device according to one of Claims 4 or 5, **characterized in that** the spring (13) has a notch (22) on an end face (20).

7. Device according to one of Claims 4 through 6, **characterized in that** a pin (21) can be inserted into an opening (23) passing through the exterior hollow body (2) and this pin (21) secures the spring (13) by a form fit with the notch (22) to prevent its rotation about the central axis (3).

8. Device according to one of Claims 1 through 7, **characterized in that** an elastic strap (24) with its free ends (25, 26) bent outward being latchable in longitudinal slits (27) on the exterior hollow body (2) and twisting of the two hollow bodies (1, 2) is prevented.

9. Device according to one of Claims 4 through 8, **characterized in that** the interior hollow body (1) is provided with a shoulder (28) on its lower end (16), the outside diameter of said shoulder being greater than the inside diameter of the unextended spring (13), preventing the interior hollow body (1) from sliding upward past the spring (13) and slipping out of the exterior hollow body (2).

10. Device according to one of Claims 1 through 9, **characterized in that** the two hollow bodies (1, 2) have through holes (30) radially to the central axis (3).

11. Device according to one of Claims 4 through 10, **characterized in that** the groove (12) and the spring (13) are designed with a conical taper toward the contact surface (31).

12. Device according to one of Claims 1 through 11, **characterized in that** one or both of the hollow bodies (1, 2) is/are cropped along the central axis (3).

13. Device according to one of Claims 1 through 12, **characterized in that** the two hollow bodies (1, 2) have a non-circular cross section.

14. Device according to one of Claims 1 through 13, **characterized in that** end plates (38, 39) are mounted on the two free ends (36, 37) of the two hollow bodies (1, 2).

15. Device according to Claim 14, **characterized in that** mandrels (40) are mounted on the end plates (38, 39) on the bone side.

16. Device according to one of Claims 1 through 15, **characterized in that** a locking screw (35) is screwed into the exterior hollow body (2).

17. Device according to one of Claims 4 through 16, **characterized in that** the catch mechanism (5; 14) is undercut in the direction of the top end (37) on the interior hollow body (1) as well as on the spring (13) on the pressure-loaded side.

## Revendications

1. Dispositif de remplacement de vertèbres, comprenant un corps creux intérieur (1) et un corps creux extérieur (2) disposés l'un dans l'autre à coulissement coaxial le long d'un axe central (3) et pouvant coulisser l'un par rapport à l'autre dans la direction de cet axe central (3), le corps creux intérieur (1) étant doté d'un chantage (5) sur sa surface d'enveloppe extérieure (4), **caractérisé en ce que**:
A) le corps creux extérieur (2) est alésé (10) de manière continue suivant l'axe central (3);
B) le corps creux extérieur (2) présente au moins un élément élastique (7) qui pénètre dans l'alésage (10) et qui diminue le diamètre de l'alésage (10) de telle sorte que l'élément élastique (7) s'engage dans le chantage (5) ménagé sur le corps creux intérieur (1); et
C) le corps creux intérieur (1) est rigide.

2. Dispositif selon la revendication 1, **caractérisé en ce que** les deux corps creux (1, 2) présentent la forme de cylindres creux.

3. Dispositif selon la revendication 1 ou 2, **caractérisé en ce que** cet élément élastique (7), au moins un, est configuré comme languette (19) découpée dans la paroi (6) du corps creux extérieur (2).

4. Dispositif selon la revendication 1 ou 2, **caractérisé en ce que** comme élément élastique (7) est prévu un ressort annulaire (13) ouvert radialement par un interstice (18), doté d'un chantage (14) sur sa périphérie intérieure (15) et monté dans une rainure périphérique (12) ménagée sur la surface d'enveloppe intérieure (11) du corps creux extérieur (2) et dans laquelle s'engage le chantage (5) ménagé sur le corps creux intérieur (1).

5. Dispositif selon la revendication 4, **caractérisé en ce que** le ressort (13) peut être écarté à l'aide d'un accessoire qui peut être inséré par l'ouverture (17) dans l'interstice (18) du ressort (13) et **en ce que** le chantage (14) du ressort (13) peut ainsi être extrait du chantage (5) prévu sur le corps creux intérieur (1).

6. Dispositif selon l'une des revendications 4 ou 5, **caractérisé en ce que** le ressort (13) présente une entaille (22) sur une surface frontale (20).

7. Dispositif selon l'une des revendications 4 à 6, **caractérisé en ce qu'**un goujon (21) est inséré par une ouverture (23) qui traverse le corps creux extérieur (2) et **en ce que** ce goujon (21) empêche le ressort (13) de tourner autour de l'axe central (3) par correspondance géométrique avec l'entaille (22).

8. Dispositif selon l'une des revendications 1 à 7, **caractérisé en ce qu'**un étrier élastique (24) présentant des extrémités libres (25, 26) rabattues vers l'extérieur peut être engagé dans des fentes longitudinales (27) ménagées sur le corps creux extérieur (2) et empêche que les deux corps creux (1, 2) tournent.

9. Dispositif selon l'une des revendications 4 à 8, **caractérisé en ce que** le corps creux intérieur (1) est doté à son extrémité inférieure (16) d'un épaulement (28) dont le diamètre extérieur est plus grand que le diamètre intérieur du ressort (13) non écarté et qui empêche que le corps creux intérieur (1) dépasse le ressort (13) en glissant vers le haut et puisse sortir du corps creux extérieur (2).

10. Dispositif selon l'une des revendications 1 à 9, **caractérisé en ce que** les deux corps creux (1, 2) présentent des ouvertures de passage (30) radiales par rapport à l'axe central (3).

11. Dispositif selon l'une des revendications 4 à 10, **caractérisé en ce que** la rainure (12) et le ressort (13) présentent une configuration conique qui se rétrécit en direction de la surface de contact (31).

12. Dispositif selon l'une des revendications 1 à 11, **caractérisé en ce qu'**un ou deux des corps creux (1, 2) présentent un gradin en direction de l'axe central (3).

13. Dispositif selon l'une des revendications 1 à 12, **caractérisé en ce que** les deux corps creux (1, 2) présentent une section transversale non circulaire.

14. Dispositif selon l'une des revendications 1 à 13, **caractérisé en ce que** des plaques d'extrémité (38, 39) sont installées sur les deux extrémités libres (36, 37) des deux corps creux (1, 2).

15. Dispositif selon la revendication 14, **caractérisé en ce que** des pointés (40) tournées vers l'os sont prévues sur les plaques d'extrémité (38, 39).

16. Dispositif selon l'une des revendications 1 à 15, **caractérisé en ce qu'**une vis de verrouillage (35) est vissée dans le corps creux extérieur (2).

17. Dispositif selon l'une des revendications 4 à 6, **caractérisé en ce que** tant sur le corps creux intérieur (1) que sur le ressort (13), le chantage (5; 14) est configuré en contre-dépouille en direction de l'extrémité supérieure (17) sur le côté sollicité en compression.
